# EUROPEAN PATENT APPLICATION

(11) **EP 1 676 587 A1**
(43) Date of publication of application: **05.07.2006**
(21) Application number: 04762299.8
(22) Date of filing: 20.10.2004
(51) Int. Cl.: A61K 39/39, A61K 39/295, A61K 39/29, A61K 39/21

(54) **PHARMACEUTICAL COMPOSITIONS FOR THERAPEUTIC USE**

(30) Priority: 20.10.2003 CU 24003
(71) Applicant: CENTRO DE INGENIERIA GENETICA Y BIOTECNOLOGIA (CIGB), Ciudad de la Habana 10600 (CU)
(72) Inventor: AGUILAR RUBIDO, Julio, César, La Habana 32 200 (CU); IGLESIAS PEREZ, Enrique, Ciudad dela Habana 11700 (CU); LOBAINA MATO, Yadira, Marti, Cerro, Ciudad de La Habana 13400 (CU); GARCIA GONZALEZ, Daymir, San Agustin, La Lisa, La Habana 13 600 (CU); MUZIO GONZALEZ, Verena, Lucila, Ciudad de La Habana 10400 (CU); GUILLEN NIETO, Gerardo, Enrique, Ciudad de La Habana 10400 (CU); ACOSTA RIVERO, Nelson, Santos Suárez, 10 de Oct., La Habana (CU); UBIETA GOMEZ, Raimundo, Ciudad de La Habana 12 100 (CU); ALVAREZ OBREGON, Julio, Cesar, Ciudad de La Habana 10 400 (CU); DUENAS CARRERA, Santiago, Plaza de la Revolucion, La Habana 10 400 (CU); DUARTE CANO, Carlos, Ciudad de la Habana 12 100 (CU); VANLANDSHOOT, Peter, B-9000 Gent (BE); HERRERA MARTINEZ, Luis, Saturnino, Ciudad de La Habana 12 100 (CU)
(74) Representative: van Loon, C.J.J.
(86) International application number: PCT/CU2004/000011
(87) International publication number: WO 2005/037311

(57) **Abstract**

The present invention is related with the field of therapeutic vaccines against pathogens causing chronic diseases. It comprises the use in therapy of formulations containing the Hepatitis B surface antigen (HBsAg) as the main compound. The HBsAg is produced as a recombinant product in *Picchia pastoris.* The formulations of the present invention also comprise the combination of the Hepatitis B surface antigen and other coadministered antigens. The resulting formulations are able to generate potent lymphoprolipherative and cytotoxic responses apart from a remarkable response of specific antibodies, which make them very effective in the treatment of such diseases.

## Description

### Field of the invention

The present invention is related with the field of Biotechnology dedicated to therapeutic vaccines against pathogens causing chronic diseases. Specifically related to the use of the Hepatitis B surface antigen (HBsAg), produced by recombinant techniques in *Pichia pastoris* as a main compound of pharmaceutic compositions for therapeutic use.

### Previos Art.

Approximately 350 million people are chronically infected by the Hepatitis B Virus (HBV). The main risk for HBV chronically infected patients is the development of cirrhosis, resulting in an increased mortality related to the hepatocellular carcinoma or non-carcinomatous complications of cirrhosis (portal hypertension and hepatic failure).

The control of viral replication reduces significantly these risks as the pathology of the HBV infection is mainly immune-mediated.

Nowadays, only two main therapies are widely recognized for treating chronic hepatitis caused by the HBV and which show clinical benefits: the treatment with α-Interferon and the therapy with the analog of nucleos/tides like Lamivudine. Both treatments have important limitations. The therapy with α-Interferon combines antiviral and immunostimulatory properties of γlFN, resulting in a sustained supression of the viral replication in only one third of patients.

Lamivudine treatment conduces to a fast and almost absolute reduction of HBV replication, but treatments for short periods of time are related to frequent relapses.

Treatments for longer periods increase the relapsing with an incidence of 15 to 20% per year. The limitations of both antiviral therapies for HBV point out the necessity to find new therapeutic afternatives, including new nucleotidic analogues and new specific or non-specific immunotherapies aimed to improve the poor responses of T cells in chronically infected patients.

The passive transfer of specific immunity, the immunomodulating drugs (citoquines, thymic derivatives and grown factors) and vaccine therapies (the current recombinant vaccines, new approaches like DNA-based vaccines and T cell peptides) are recent immunotherapeutic strategies.

The HBV is not a cytopatic virus and the hepatic damage during the infection is mostly mediated by the host immune response *vs*. the infected cells and due to the production of inflammatory cytokines. A potent, polyclonal and multispecific cellular response in the subsets of cytotoxic and helper cells as well as a strong cytoquine response *vs*. the HBV is easily and rapidly detectable in peripheral blood of patients with acute and self-limited Hepatitis B infection. In turn, patients with chronic infection, the same response is weak, antigenically restricted or undetectable. It has been demonstrated that the T cell response is responsible of the HBV elimination.

During acute hepatitis B, Th1 cytoquines (γ-interferon, TNF-α) and IL-2 have been related to viral clearance.

Several recombinant anti Hepatitis B vaccines have been assayed for immunotherapy. The immunization of transgenic mice expressing constitutively the HBsAg in the liver showed the capacity of this approach to overcome the functional tolerance to HBsAg inducing a specific immune response (Mancini M, et al., Induction of anti-hepatitis B surface antigen (HBsAg) antibodies in HBsAg transgenic mice: a possible way of circumventing "nonresponse" to HBsAg. J Med Virol. 1993; 39:67-74.). Initial clinical trials in pilot scale evidenced that specific vaccine therapy was able to eliminate or to reduce the replication of the HBV in appr. 50% of the chronic carriers (Pol S, et al. Lancet 1994; 342 (Letter), Wen Y-M, et al. Lancet 1995; 345: 1575-1576 (Letter), Pol S, et al. Acta Gastroenterologica Belgica 1998; 61:228-33).

Finally, a multicentric and controlled study showed the actual capacity of anti-hepatitis B vaccines in the treatment of chronic infection (Pol S, et al. J Hepatol 2001; 34: 917-21). Commercial vaccines containing the antigen including pre-S regions or with the S region alone were assayed. After one year and five injections there was no difference in the percent of negativization of viral DNA compared to the non-treated control group, there was no clearance of HBsAg and the negativization of the "e" antigen was also poor though favorable to the vaccine group. All these results evidenced the need for new immunotherapeutic strategies as well as to conclude that the reinforcement of immunization strategies with more potent candidates is a need of the previously described strategy.

It was recently evidenced that the antigens with lower capacity to bind CD14 receptor were able to induce a higher proliferative activity in PBMC of individuals who generated a high response to HBsAg. This fact suggests a correlation between the capacity to bind CD14 with an immunosuppressive mechanism (Valandschoot P, et al. J. Med Virol 2003, 70: 513-519).

A new Hepatitis B surface antigen, evidencing its superior immunogenicity in terms of cellular responses, which demonstrated enhancing activity on co-administered homologous and heterologous antigens and also produced by a process which selects a variant with a significant reduction in its binding capacity to the CD14 receptor -as a result of its physico-chemical characterization- is a novel antigen and can be considered to be used in the antiviral therapy against the Hepatitis B Virus, alone or included as part of formulations of multiple antigens for the treatment of chronic infections or co-infections.

### Detailed description of the invention

In the present invention, it has been described a new use of the Hepatitis B surface antigen produced in *Pichia pastoris* by a method described in this application, to obtain new pharmaceutical compositions for therapeutic use. In order to achieve this, it has been taken into consideration the new properties of the HBsAg produced in our conditions compared with other Hepatitis B antigens.

In the present invention we have found that the method described in the patent EP 0480525 B1 "Method for obtaining recombinant surface antigen of Hepatitis B Virus" provide new properties to the resulting antigen. The HBsAg produced in *Pichia pastoris,* using a purification process which comprises the following steps: cell lysis in a lysis buffer containing a caotropic agent; precipitation of contaminants in acidic conditions (low pH values); immunoaffinity chromatography of the antigen using a specific monoclonal antibody against the Hepatitis B surface antigen; thermal treatment of the eluted antigen with temperatures ranging from 30°C to 40°C; results in a new antigen which is different from other Hepatitis B surface antigens described in literature, supporting the therapeutic use of this antigen.

The differences among HBsAg antigens are based in the higher immunogenicity of the antigen subject matter of the present invention compared to the antigens produced in other hosts and using different purification methods. Another difference is the enhancing activity on homologous and heterologous antigens also considered as potential antigens to be included in therapeutic vaccines to be co-administered as a result of the enhancing activity of our HBsAg. Furthermore, there are different aspects of the structure and chemical composition of the HBsAg produced under our conditions which markedly influences in their behavior in front of the immune system.

The HBsAg of the present invention can be used in vaccine formulations to treat Hepatitis B chronic infection and also in cases of chronic co-infection with other viruses due to the capacity of the antigen to promote an enhancement of the cellular response, prolipherative and functional against the heterologous antigens.

Among the antigens that have been co-administered along with the HBsAg of the present invention, there are antigens from other viruses, able to generate chronic infections, for example Hepatitis C and HIV antigens. The results described in the examples of the present invention have been obtained with the nucleocapsid of the Hepatitis C Virus and with a chimeric protein of VIH-1 containing epitopes related to the cellular response in humans against said pathogens.

This property, according to the evidences we have obtained in our lab, is extensive to any homologous or heterologous protein that -with the same objective- could be co-administered with the HBsAg obtained from this process. An homologous protein that can be used in our formulation and also important to the final effect of the formulation, is the protein of the nucleocapside of the Hepatitis B virus or HBcAg. This protein is able to receive an immunoenhancing effect in its cellular response when it is co-administered along with the HBsAg of the present invention.

At the same time, it is obvious from this invention the possible use of the resulting multivalent formulations in individuals suffering the chronic course of a disease apart from Hepatitis B that will be benefited therapeutically against the chronic disease and at the same time they will induce a preventive immune response against the infection by the HBV infection.

Also a subject matter of the present invention is the pharmaceutic composition using the HBsAg obtained in Pichia *pastoris* using the method described for the therapy against chronic Hepatitis B, both in solution as well as conveniently adjuvated in alum salts or any other adjuvant selected with this objective. They could be alum salts or any other adjuvant useful in the administration of our vaccine for therapeutic use but they are not restricted to them.

It is also a subject matter of the present invention, a Pharmaceutical composition for therapeutic use in which the Hepatitis B surface antigen obtained in *Pichia pastoris* by the described method is administered in combination with homologous and heterologous antigens that will receive an important adjuvant effect for their therapeutic activity. The homologous antigen is comprised among the antigens of the Hepatitis B Virus, preferentially the nucleocapside antigen of said virus. Other antigens that could be included in formulations subject matter of the present invention are those belonging to viruses with a chronic evolution in humans, for example the Hepatitis C virus and the HIV-1, but not restricted to them, enabling the use of antigens from other viruses with such characteristics.

It is important to point out that the pharmaceutical compositions of the present invention can be used through the mucosal, parenteral or combining both routes aimed to reinforce the kind of therapeutic response required. The amount of antigen in the pharmaceutic compositions of the present invention will depend of the antigens involved in the formulations, in the case of HBsAg; the antigen is in the range between 5 and 300 micrograms per dosis. The proportion to the rest of the coadministered antigens varies from 5:1 to 1:5, depending on the specific treatment to be considered.

### Description of Figures

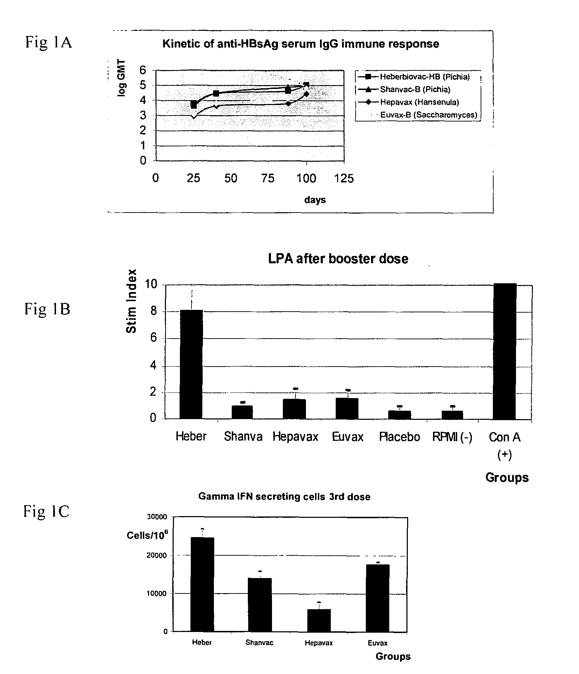

**Figure 1.** 1A) Graphic showing the kinetic of the anti HBsAg IgG immune response in the sera of mice immunized on days 0, 15, 30 y 90, with different commercial vaccines. One of the vaccines is Heberbiovac-HB, which contains the HBsAg obtained in *Pichia pastoris* and other vaccines produced in other hosts and with different methods. (1 B) Lymphoprolipherative response generated by the different vaccines after the booster dose on day 90.1C) Response of γlFN secreting cells after stimulation with cells of murine mastocytome p815 pulsed with the peptide S₂₈₋39.

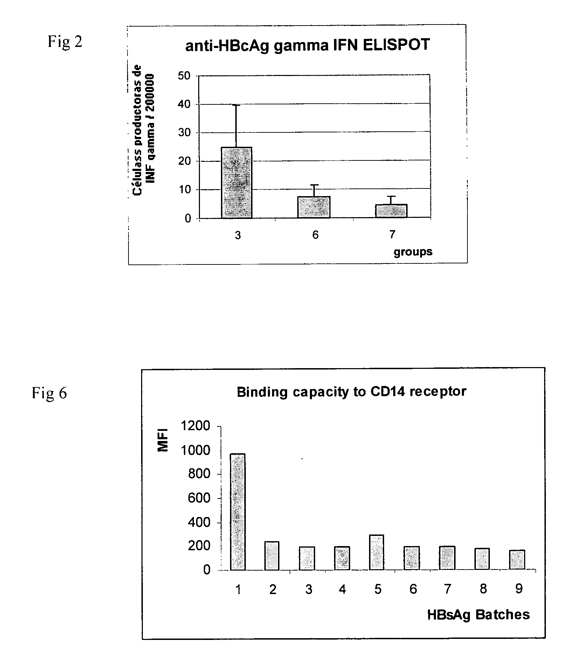

**Figure 2.** Comparative study of the antigen produced by *Pichia pastoris* and the antigen produced in cells from superior organisms (CHO) in respect to their capacity to enhance the cellular response against other co-administered antigens. In this case the Hepatitis B core antigen was used as an antigenic model.

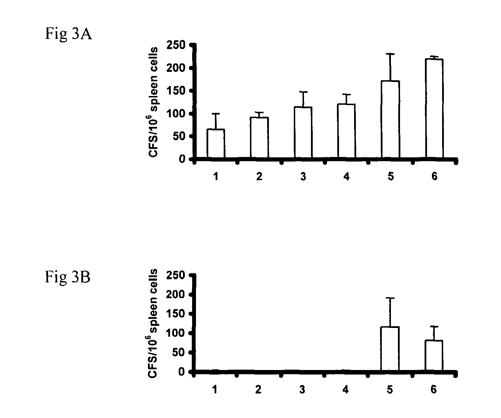

Figure 3. Study of the immune response induced by combined formulations for therapeutic use, comprising the HBsAg, the HBcAg and a multiepitopic protein containing sequences from HIV-1. Nasal immunisation schedule: 3A) Anti-CR3 CD8+ lymphocytes secreting γlFN after stimulation of spleen cells with the transgenic p815 with the sequence of CR3 inserted. 3B) γlFN secreting lymphocytes after direct stimulation of spleen cells using CR3.

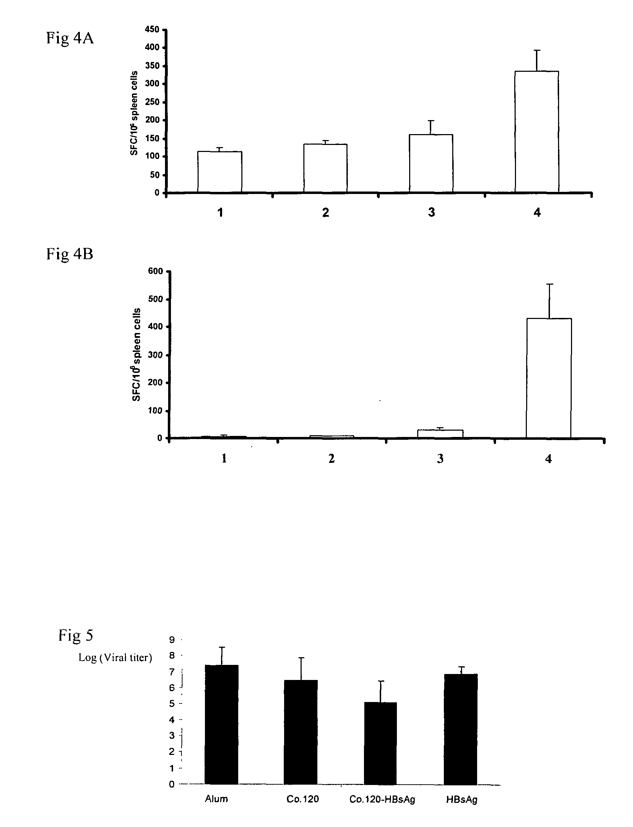

Figure 4. Study of the immune response induced by combined formulations of the HBsAg, HBcAg and the CR3 protein from HIV-1. Parenteral immunisation schedule. 4A) Anti-CR3 CD8+ lymphocytes secreting γlFN after stimulation of spleen cells with the transgenic p815 with the sequence of CR3 inserted. 4B) γlFN secreting lymphocytes after direct stimulation of spleen cells using CR3.
Figure 5. Viral titer in ovaries of animals from different groups.
Figure 6. CD14 binding capacity group 1) HBsAg produced in Saccharomyces groups 2-9) HBsAg produced *in Pichia* in the described conditions.

### EXAMPLES

### Example 1: Immunogenicity of different Hepatitis B vaccines produced from different hosts regarding their ability to induce humoral and cellular immune responses.

Aimed to study the humoral and cellular immune responses generated by different commercial anti-hepatitis B vaccines, an immunisation schedule was carried out in Balb/C mice. Five groups of 10 mice each, from 8 to 12 weeks of age were immunized on days 0, 15, 30 and 90 with a dose of 2µg (micrograms) of HBsAg intramuscularly. Blood extractions were conducted 10 days after each administration in order to evaluate the humoral response and after third and fourth dose mice were sacrificed in groups of 3 to 4 animals per group to study the immune response depending on the type of study.

In this study, the HBsAg dose of 2µg per mice was selected as it is low enough to enable the detection of any difference between very similar products (all assayed vaccines have similar amounts of HBsAg and alum hydroxide).

The groups of the immunisation schedule were 1) the Heberbiovac HB vaccine containing the HBsAg antigen produced in *Pichia pastoris,* 2) the Shanvac B vaccine, also produced in *Pichia pastoris* but using other purification method, 3) Hepavax Gene and 4) the Euvax B vaccine produced in *Saccharomyces cerevisiae* and *Hansénula polymorpha* respectively. A fifth group was the placebo control comprising the mice receiving only the alum hydroxide in similar amount.

The humoral immune response, as well as the duration of titers was evaluated by ELISA determining total IgG in serum samples. Serum IgG titers were tested two weeks after the second, third and fourth doses and also previous to the fourth dose. Results of the antibody response were shown in figure 1A.

During the analyzed period of time, no significant differences were found between groups immunized with the Heberbiovac HB and Shanvac B vaccines. Both vaccines generated similar titers and superior to the rest of the assayed vaccines (Euvax B y Hepavax Gene) after the second and third dose.

Ten days after the fourth dose, 4 mice per group were sacrificed to conduct studies of cellular immunity measuring the lymphoproliferative responses per individual mice. In our experimental conditions of low antigenic dose only the Heberbiovac HB was able to induce a lymphoproliferative response clearly detectable after the booster dose. There was no positive response for the rest of the vaccines under study (fig. 1 B).

The lymphoproliferative assay indicates the capacity of spleen cells to proliferate without a selection of specific cell subsets, but it is known for the time of the assay that the memory T CD4+ is the main cell population responding to the stimulus. This T CD4+ lymphocytes proliferating specifically to the antigen stimulus can interact with B cells and with CD8⁺ T cells helping in the development of humoral and cytotoxic responses respectively.

Ten days after the third dose, three mice per group were sacrificed and ELISPOT assay was conducted, the results are shown in figure 1C.

The results obtained in ELISPOT assay evidenced that the HBsAg in alum is able to induce a strong response of γlNF secreting lymphocytes after restimulation with the S₂₈₋₃₉ peptide derived from HBsAg. A direct ELISPOT assay evidenced lower responses compared to the results after restimulation (data not shown). The results shown in figure 1C evidenced that Heberbiovac HB has the ability to induce a higher frequency of γlFN secreting cells in spleen cells from animals immunized compared to the rest of vaccines under evaluation. The secretion of γIFN indicates the development of Th1 cells, which is highly important in the control of the HBV replication.

Taken together the results obtained demonstrates the superior cellular response of the HBsAg produced in *Pichia pastoris* and purified in the conditions explained in this invention respect to the antigens obtained in other hosts as well as in the same host and different conditions. This result is remarkable in the field of therapeutic vaccination as it is widely accepted the correlation between the responses evaluated in this experiment (lymphoproliferative activity and γlFN secretion) with the control of Hepatitis B viral infection.

### Example 2: Enhancing effect on the cellular immune response by the HBsAg produced by the described method.

In order to evaluate the potentialities of the HBsAg obtained from *Pichia pastoris* respect to the HBsAg antigen obtained in other hosts, an experiment to evaluate the immune response induced by the co-administration of both antigens on the homologous antigen HBcAg (the hepatitis B nucleocapsid antigen) was planned.

With this purpose, it was evaluated the frequency of γIFN secreting cells after stimulation of spleen cells with the nucleocapsid antigen by ELISPOT assay.

The determinations were carried out in groups immunized with the HBsAg from Heberbiovac HB and also with the HBsAg produced from superior organism (CHO), in both cases co-administered with the nucleocapside antigen. Furthermore, the corresponding placebo controls were evaluated (data not shown) as well as the control containing the nucleocapsid antigen alone (group C, fig. 2).

This result evidenced an increase in the frequency of cells secreting γlFN in the group immunized with the HBsAg produced from *Pichia pastoris* (group A) respect to the group containing the HBsAg produced in CHO cells (group B, fig. 2). Then, we can propose the use of the HBsAg produced from *Pichia* in the design of therapeutic formulations including other antigens: homologous as in this case or heterologous as we will show in further experiments.

### Example 3: Use of HBsAg in therapeutic vaccines formulations combining HBV antigens and HIV-1 derived protein CR3 from HIV-1.

To evaluate the use of HBsAg in nasal immunizations as an antigen and at the same time as an adjuvant for the immunopotentiation of cell specific responses against heterologous antigens in therapeutic vaccine formulations, six groups of 6-8 weeks old female Balb/c mice were inoculated following the schedule 0, 7, 14, 35 and 63. The animals were immunized in different groups by the intranasal route as follows: 1. PBS, 2. HBcAg + HBsAg, 3. CR3, 4. HBcAg + CR3, 5. HBsAg + CR3, 6. HBcAg + HBsAg + CR3. The CR3 antigen corresponded with a multiepitopic polypeptide obtained by recombinant technologies and containing several sequences from HIV-1, specific for human T cells. The antigens were dissolved in PBS and dispensed in 50µl (25µl per nostril). The doses of 5, 5 and 10 µg per mice were used for HBsAg (CIGB, Cuba), HBcAg (CIGB, Cuba) y CR3 (CIGB, Cuba), respectively. The specific anti-CR3 γlFN secreted immune responses were measured in ELISPOT assays for CD8+ cells as well as using total spleen cells 10 days after the last inoculation. The study of cellular response was carried out by measuring the frequency of spleen cells secreting γlFN by ELISPOT assay. This APC produces constitutively the CR3 protein, which is intracellularly processed and presented in HLA class I. The mastocytome P815 do not express the MHC-II molecules in their surface, due to this fact the P815CR3 will specifically activate the CD8+ T cells. It was evidenced that only the groups immunized with HBsAg + CR3 and HBcAg + HBsAg + CR3 were positive responders (Fig. 3A) demonstrating the enhancing activity on anti CR3 cellular response stimulated by the HBsAg on the CR3 protein. Using total spleen cells, the protein CR3 was added to the culture where it is endocyted and processed to be presented mainly in the context of MHC class II of professional APC, like macrophages and dendritic cells.

Our results showed that only the same groups presenting a positive response to the CD8+ T lymphocytes were also positive to the above described assay (using total spleen cells), where the main responders were CD4+ cells: HBsAg + CR3 and HBcAg + HBsAg + CR3 (Fig. 3B). In general, it was possible to conclude that the HBsAg enhanced the cellular response against HIV-1 as a result of potentiating the response against their epitopes presented by the CR3 protein. This result justifies the use of the HBsAg as an antigen as well as an adjuvant in the context of therapeutic immunization against the HIV-1 and HBV at the same time.

### Example 4: Use of HBsAg in therapeutic vaccines combined to immunogens from HIV-1 through parenteral route.

In order to evaluate the use of HBsAg in parenteral immunizations as an antigen and as well as an adjuvant of cellular responses in the field of combined HBV-HIV immunizations, different formulations were assayed. Groups of 8 to 10 female Balb/c mice (CENPALAB, Cuba), of 6-8 weeks old were immunized on days 0, 14, 35. Mice were immunized subcutaneously in different groups as follows: 1) HBsAg, 2) CR3, 3) HBsAg + CR3, 4) HBsAg + HBcAg + CR3. All the immunogens were adjuvated in 1 mg/mL alum phosphate. The following HBsAg and HBcAg doses were used: 2, 4 and 10 µg in 100 µl per mice. Both antigens were produced at CIGB, Cuba. The anti-CR3 specific cellular immune response of γlFN secreting cells was measured by ELISPOT using the P815 expressing CR3 as described in example 3 (Fig. 4A). Positive responses were only detected in the case of groups immunized with: HBsAg + CR3 and HBcAg + HBsAg + CR3 in alum phosphate (Fig 4A) evidencing the effect of the HBsAg on the immunogenicity of the co-administered antigen.

The analysis of the anti-CR3 specific cellular immune response of γlFN secreting cells was carried out also when the CR3 protein was added directly to the culture without any APC like P815. This experiment resulted in a positive ELISPOT response only for the same groups which resulted positive in the case of the CD8+ response. They are: HBsAg + CR3 and HBcAg + HBsAg + CR3 in alum phosphate (Fig. 4B), these groups evidenced again the cellular enhancing activity of HBsAg on CR3.

### Example 5: Study of the combination of HBsAg and HCV antigens.

With the aim of evaluating the influence of combining the truncated protein from the HCV capsid (Co.120), (Lorenzo LJ, et al. Biochem Biophys Res Commun 2001 ;281 (4):962-965) and the HBsAg on the immune response generated vs the antigens of the structural region from HCV, an intramuscular schedule was carried out in BALB/c mice. Groups of 10 female mice of 8 weeks old were used. The schedule included four inoculations on weeks 0, 3, 6 y 9. Blood extractions were conducted 11 weeks after the first immunization. All the immunogens were adjuvated in alum hydroxide. Group 1 was used as a negative control and received only the alum hydroxide. Group 2 was inoculated with 5 µg of the protein Co.120. Group 3 was inoculated with the mixture of 5 µg of each protein and the group 4 was immunized with 5 µg of HBsAg. Animals were challenged two weeks later through the peritoneal route administering 10⁶ plaque forming units of a recombinant vaccinia virus with the HCV capsid antigen inserted by recombinant technologies.

Animals were sacrificed 5 days after challenge and the ovaries were extracted and processed. After serial dilutions, BSC-40 cells were infected during 2 days in order to determine the viral titer using Crystal violet staining.

Figure 5 shows the viral titer in ovaries from different groups. The student T test was employed in the statistical analysis, comparing the results between each group and the control one, being considered p<0.05 as a significant difference. In figure 5, it is shown that immunized animal with the mixture of Co.120 protein and HBsAg have a significantly lower viral titer as compared with the rest of the groups (p<0.05), evidencing the induction of a strong and effective cellular response in vivo against the capsid of the Hepatitis C Virus. This group was the only group able to significantly reduce the viral titer respect to the negative control group (mice immunized with alum hydroxide). Hence, it was demonstrated in this system of direct in vivo assay of functional antiviral capacity, the enhancing activity of HBsAg in term of viral titer reduction.

### Example 6: Study of the enhancing effect of the HBsAg described in this application on different antigens associated to several pathologies.

Aimed to evaluate the use of HBsAg in parenteral immunizations as an antigen and at the same time as an immunoenhancer of the specific cellular immune response in the field of virus related to chronic diseases, different experiments were conducted using formulations containing the HBsAg and different coadministered antigens in the same preparation. The coadministered antigens comprised the antigens from human pathologies like: HIV (attenuated viruses containing antigens from the HIV-1 virus, peptides and multiepitopic peptides); HPV (we have worked with virus-like particles from the HPV 16, 18 and 33; HCV (antigens containing sequences from the nucleocapsid, peptides and proteins from their surface antigens, as well as segments from non-structural sequences; similarly, antigens from other pathologies generating chronic diseases like cancer (tumor associated and tumor specific antigens from lung, liver, breast and prostate). In all cases it was appreciated an increase in the γlFN secreting activity in respect to the administration of the antigens alone, demonstrating the potentialities of the HBsAg described in our application to be administered in therapeutic formulations.

### Example 7. Study of the HBsAg capacity to bind the CD14 receptor using different batches of HBsAg obtained from Pichia pastoris, using the described method of production compared to the antigen obtained in other hosts.

The analysis of the HBsAg obtained as a recombinant protein using the purification method described in the present invention evidenced that there are differences influencing the higher immunogenicity at cellular compartment as it is the case of the binding capacity of HBsAg to the CD14 receptor found in macrophages and related to the development of processes of immunological tolerance.

It was probed, after immunocytometric analysis that there is a marked reduction in the capacity of binding the CD14 receptor for the antigen produced in the yeast *Pichia pastoris* produced in the conditions described in this application compared to the antigen produced in *Saccharomyces cerevisiae* (Fig. 6). This fact supports our finding of differential behavior of the antigens assayed in the present invention.

## Claims

1. Pharmaceutic compositions for therapeutic use **characterized by** the inclusion of the Hepatitis B surface antigen produced from yeast by a purification method comprising at least one of the following steps: cell lysis in a lysis buffer containing a caotropic agent; precipitation of contaminants in condition of acid pH; immunoaffinity chromatography of the antigen using a specific monoclonal antibody against the Hepatitis B surface antigen; thermal treatment of the eluted antigen to temperatures ranging from 30°C to 40°C.

2. Pharmaceutic compositions for therapeutic use according to claim 1 wherein the yeast used for antigen production is *Pichia pastoris.*

3. Pharmaceutic compositions for therapeutic use according to claims 1 and 2 **characterized by** their use as treatments for chronically progressing viruses.

4. Pharmaceutic compositions for therapeutic use according to claims 1 to 3 **characterized by** their use as treatments for the chronic infection by the Hepatitis B Virus.

5. Pharmaceutic compositions for therapeutic use according to claims 1 to 3 **characterized by** their use as treatments for the chronic co-infection by the Hepatitis B Virus and the Hepatitis C Virus.

6. Pharmaceutic compositions for therapeutic use according to claims 1 to 3 **characterized by** their use as treatments for the chronic infection by the Hepatitis B Virus and the Human Immunodeficiency Virus.

7. Pharmaceutic compositions for therapeutic use according to claims 1 to 3 **characterized by** their use as treatments for the chronic infection by the Hepatitis B Virus and other infection comprised in the group of chronic infectious diseases.

8. Pharmaceutic compositions for therapeutic use according to claims 1 to 3 **characterized by** their use as bivalent vaccines to be used in the treatment of a chronic disease and at the same time as a preventive vaccine for the Hepatitis B Virus.

9. Pharmaceutic compositions for therapeutic use according to claims 1 to 8 wherein the Hepatitis B surface antigen is administered alone or conveniently adjuvated.

10. Pharmaceutic compositions for therapeutic use according to claims 1 to 9 wherein the Hepatitis B surface antigen is administered combined to several homologous and heterologous antigens that will receive an adjuvant effect from the HBsAg of remarkable importance for their therapeutic activity.

11. Pharmaceutic compositions for therapeutic use according to claim 10 wherein the heterologous antigen can be a vaccine antigen from a virus which develops a chronic disease.

12. Pharmaceutic compositions for therapeutic use according to claims 1 to 11 for parenteral use, mucosal use or used combining both routes.

13. Use of the Hepatitis B Surface Antigen in pharmaceutical compositions for therapeutic use wherein the antigen is produced in yeast through a purification method comprising at least one of the following steps: cell lysis in a lysis buffer containing a caotropic agent; precipitation of contaminants in condition of acid pH; immunoaffinity chromatography of the antigen using a specific monoclonal antibody against the Hepatitis B surface antigen; thermal treatment of the eluted antigen to temperatures ranging from 30°C to 40°C..

14. Use of the Hepatitis B Surface Antigen according to claim 13 wherein the yeast employed for the production of the antigen is *Pichia pastoris.*

15. Use of the Hepatitis B Surface Antigen according to claim 13 and 14 to obtain a vaccine for the treatment of an infection caused by a Virus of chronic evolution.

16. Use of the Hepatitis B Surface Antigen according to claim 13 to 15 to obtain a vaccine for the treatment of the chronic infection caused by the Hepatitis B Virus.

17. Use of the Hepatitis B Surface Antigen according to claim 13 to 15 to obtain a vaccine for the treatment of the chronic co-infection caused by the Hepatitis B Virus and the Hepatitis C Virus.

18. Use of the Hepatitis B Surface Antigen according to claim 13 to 15 to obtain a vaccine for the treatment of the chronic co-infection caused by the Hepatitis B Virus and the Virus of Human Immunodeficiency-1.

19. Use of the Hepatitis B Surface Antigen according to claim 13 to 15 to obtain a vaccine for the treatment of the chronic co-infection caused by the Hepatitis B Virus and other infection comprised in the group of diseases of chronic evolution.

20. Use of the Hepatitis B Surface Antigen according to claim 13 to 15 to obtain a bivalent vaccine for the treatment of the chronic infection caused by a virus of chronic evolution and at the same time with a capacity to prevent the infection by the Hepatitis B Virus.

21. Method to treat infections caused by chronically progressing viruses **characterized by** the use of the Hepatitis B surface produced by the yeast using a method of purification comprising at least one of the following steps: cell lysis in a lysis buffer containing a caotropic agent; precipitation of contaminants in condition of acid pH; immunoaffinity chromatography of the antigen using a specific monoclonal antibody against the Hepatitis B surface antigen; thermal treatment of the eluted antigen to temperatures ranging from 30°C to 40°C.

22. Method according to claim 21 **characterized by** the use of the yeast *Pichia pastoris* to produce the HBsAg.

23. Method according to claims 21 and 22 to treat the chronic infection caused by the Hepatitis B Virus.

24. Method according to claims 21 and 22 to treat the chronic co-infection caused by the Hepatitis B Virus and the Hepatitis C Virus.

25. Method according to claims 21 and 22 to treat the chronic co-infection caused by the Hepatitis B Virus and the Virus of the Human Immunodeficiency-1.

26. Method according to claims 21 and 22 to treat the chronic co-infection caused by the Hepatitis B Virus and other infection comprised in the group of diseases of chronic evolution.

27. Method according to claims 21 and 22 to obtain a bivalent vaccine to treat the chronic infection with a virus of chronic evolution and at the same time with the capacity to prevent the Hepatitis B Virus infection.
